# EUROPEAN PATENT APPLICATION

(11) **EP 0 927 549 A1**
(43) Date of publication of application: **07.07.1999**
(21) Application number: 97204095.0
(22) Date of filing: 30.12.1997
(51) Int. Cl.: A61F 5/00, A61F 5/448

(54) **Adapter with floating flange for use with a two-piece stoma system**

(71) Applicant: Eurotec Beheer B.V., 4705 AJ Roosendaal (NL)
(72) Inventor: van der Leden, A. G., 2950 Kapellen (BE)

(57) **Abstract**

An adapter for a 2-piece stoma system, incorporating a new low profile coupling system, existing of two flat circular shaped coupling members of which the smaller coupling member has an aperture in the centre, whereby the inner circumference is completed with an inwardly extending deflectable seal strip and the larger coupling member has an aperture in the centre encircled by a projecting rib coupling member, whereby these two coupling members are joined together by a continuous relatively thin flexible polymeric wall.

The aperture in the smaller coupling member is sized to fit securely around the projecting rib coupling member of a body side flange, whilst the projecting rib on the larger coupling member is sized to fit securely within the aperture of a low profile coupling member with a deflectable seal strip on a 2-piece stoma pouch.

## Description

A number of diseases involving the intestinal and urinary tracts may result in the construction of an artificial outlet on the abdominal area for stool and/or urine.

In the case of diversion of the large intestine into the abdominal wall, one talks about a colostomy. In the case of diversion of the small intestine one talks about an ileostomy. Thirdly in the case of an urostomy, the urine is disposed through an artificial opening in the abdominal wall. The artificial openings of a colostomy, ileostomy and a urostomy are collectively referred to as stomas. During the past years a large variety of stoma appliances have been developed.

Patients with a colostomy collect the relatively normal faeces in closed pouches, which usually are changed several times a day. Patients with an ileostomy collect their relatively liquid stool in drainable pouches, the opening of which can be opened and closed with a tail clip. Patients with an urostomy collect their urine in a pouch with a drain tap. Ileostomy and urostomy pouches are usually changed once a day.

Stoma appliances are to be divided in two main systems; the 1-piece and the 2-piece system. In case of a 1-piece system, the body side wafer - which consists of a special adhesive - is integral with the pouch. This type of pouch is designed to be affixed directly to the skin, and has the advantage that the total surface of the adhesive is flat and flexible, which allows it to follow the contours of the skin. However, the major disadvantage of an 1-piece system is the fact, that the adhesive has to be removed from the skin with every pouch change, which may cause skin irritations.

In case of a 2-piece stoma system, the body side adhesive and the pouch are two separate components. First one affixes a special wafer and flange on to the skin. This flange is usually made from plastic and has a circular shape with an upstanding or projecting rib as a coupling member. The stoma pouch is also provided with a circular shaped coupling member, which can be attached on or around the projecting rib of the body side flange.

A major advantage of a 2-piece stoma system is that the peri-stomal skin will not be disturbed during pouch changes, as the adhesive part will stay in place.

A major disadvantage of the existing 2-piece stoma systems is that the body side adhesive wafer becomes less flexible, because of the rigidity and the higher profile of the in-built coupling member which makes it less suitable for stomas positioned in difficult areas such as folds and irregularities of the skin.

With both 1-piece and 2-piece stoma systems problems may occur in the post-operative period, when the wound is still fresh and the peristomal skin is very sensitive for pushing, pulling and sliding forces. In case of a 2-piece system an extra problem may occur, caused by a weak abdominal musculature, whereby the muscles are not able to give enough resistance, so that the attachment of the pouch on to the body side flange needs too much pressure, which may cause discomfort.

Therefor the prior art has sought appliances which allow both the attachment and removal of pouches without burdening the sensitive or weak peristomal skin.

With that objective in mind, Salt and Gerrard have disclosed a design for a stoma adapter in British patent application 1274382, to be used in between the abdominal skin and an 1-piece stoma pouch, whereby the surface which adheres to the skin and the surface that adheres to an 1-piece stoma pouch are only connected by a circular weld around the stoma aperture, so that a kind of collar exists. Thus one could change 1-piece stoma pouches without disturbing the sensitive skin.

Hollister describes in a patent application in 1982 - NL patent application 82022929 - a 2-piece stoma system, whereby the circular coupling member is not bonded directly to the body side wafer, but it has been bonded to a floating flexible polymeric wall, permitting the patient to insert the finger tips beneath the two coupling members, so that the connection of this 2-piece coupling system can be achieved by pressing both coupling members together between the finger tips and thumbs without pressure on the sensitive peri-stomal skin.

Also the patent application of Jensen - European patent application 0098718 from1983 - is based upon a similar principle.

In order to reduce the problems with the pressure forces when attaching a pouch of a 2-piece system on a sensitive or weak abdomen, Arnone and Ferguson have filed a patent application in 1984 - European Patent application no. 0145161 - concerning an adapter which is based on the 2-piece stoma system designed by Steer et al (British patent application 571,657). This stoma system consists of a body side wafer produced from a special hydrocolloid adhesive, covered by a thin polyethylene film, on which a circular shaped polymeric flange with an upstanding or projecting rib has been affixed, which functions as a coupling member. On to this coupling member a stoma pouch can be attached, which channel shaped coupling member is sized to snap securely over the projecting rib on the body side flange. This way of attaching a 2-piece stoma pouch may cause pressure on the sensitive skin, which - as mentioned before - can be very undesirable.

The 2-piece stoma system developed by Steer et al, can be characterised by its property that all stoma appliances and stoma pouches within this system are provided with a polymeric circular channel shaped coupling member, sized to snap over the projecting rib on the body side flanges.

The projecting rib fits exactly in between the two walls of the channel in the coupling member of the stoma pouch or stoma appliance.

With such kind of a connection the total profile of the two connected couplings as a whole will be higher than that of the profile of each separate coupling member.

This may be a disadvantage as this higher profile can be visible under tight clothes and also reduces the flexibility of the system when bending and moving the body.

The above mentioned adapter of Arnone and Ferguson also includes such a circular polymeric channel shaped coupling member, sized to snap over the projecting rib on the coupling member of the body side wafer, as this adapter is also based on the Steer at al 2-piece stoma system.

It therefor suffers from the same disadvantages.

The channel shaped coupling member and the opposite larger rib shaped coupling member of the Arnone/Ferguson adapter are joined together by a continuous relatively thin flexible polymeric wall, including a couple of accordion folds.

When the channel shaped coupling member of that adapter is attached to the body side wafer, the rib shaped opposite coupling member in its turn is sized to fit into channel shaped coupling member of a stoma pouch or stoma appliance.

Especially in case of an adapter - which in fact forms an extra part in between the body side wafer and the pouch - it is extremely important that the total profile of the two connected couplings as a whole is not higher than that of the profile of each separate coupling member.

Another important factor is, that we believe that the continuous polymeric wall between the two coupling members of the adapter need to be not too large and preferably flat without any folds.

If this wall is relatively long and provided with accordion folds, it will allow the stoma pouch to hang over when it becomes more heavy due the stool or urine, which makes the total contours of the system even more visible under the clothes. We believe that a straight smooth wall is also preferable from a hygienic point of view, as this type of wall can be cleaned much easier because no remains of stool can be trapped between the folds.

The invention described in this patent application is based on a different 2-piece coupling system, whereby the adapter and the stoma pouches are sized to fit the earlier described projecting rib of the body side coupling member, however not by using a channel shaped coupling member, but a by means of a flat circular shaped preferably solid polymeric disk with a centralised stoma aperture, which inner circumference is completed with an inwardly extending deflectable seal strip.

This relatively flat type of a coupling member is joined together with a larger circular polymeric coupling member with an outward projecting rib, by means of a continuous relatively thin flexible polymeric wall optionally provided with one or more accordion folds.

The round aperture in the smaller flat circular coupling member can be fitted securely around the outer wall of the projecting rib on the flange of the body side wafer. This can be done on a table prior to use, so that unnecessary pressure on the sensitive or weak skin can be avoided.

Subsequently, the combined body side wafer connected with an adapter can be smoothly adhered to the sensitive skin. Subsequently a stoma pouch or stoma appliance provided with the same low profile circular coupling member can be attached securely around the outer wall of the projecting rib on the largest coupling member of the adapter.

This can be achieved by pressing the two coupling members together between the finger tips and thumbs, so that the sensitive underlying skin does not suffer from any pressure.

In order to make this adapter more universal in use, the diameters and profiles of this 2-piece system are not only designed to fit the above mentioned low profile coupling members, but they are also sized to fit the channel shaped coupling members of stoma pouches, described by Steer et al.

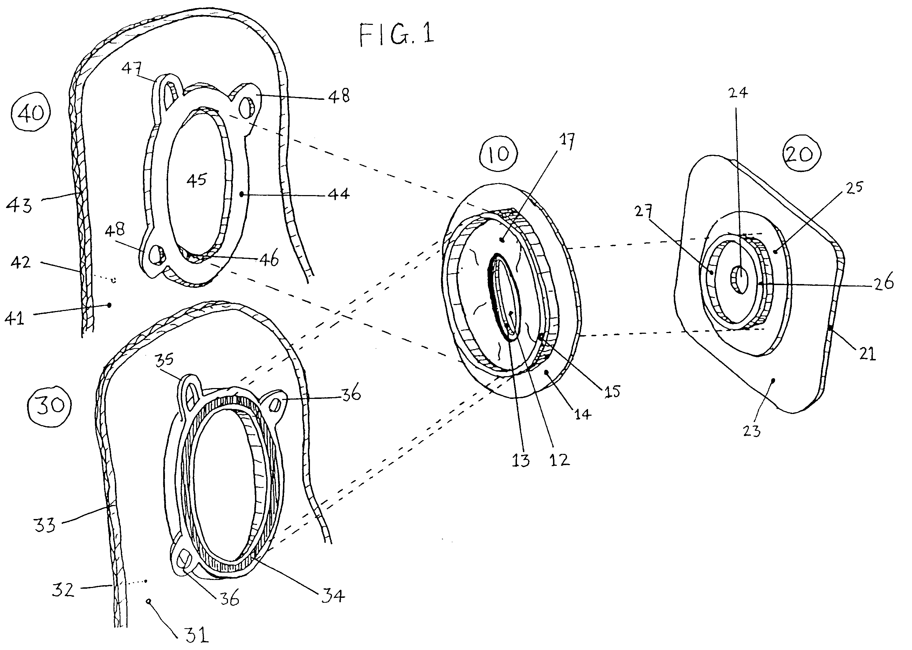

Fig. 1. Shows this adapter (10) in the centre of the drawing in between the body side wafer (20) with projecting rib (26) and two types of stoma pouches (30) and (40), which - although their coupling members have a totally different kind of coupling system - both fit well on to the same adapter.

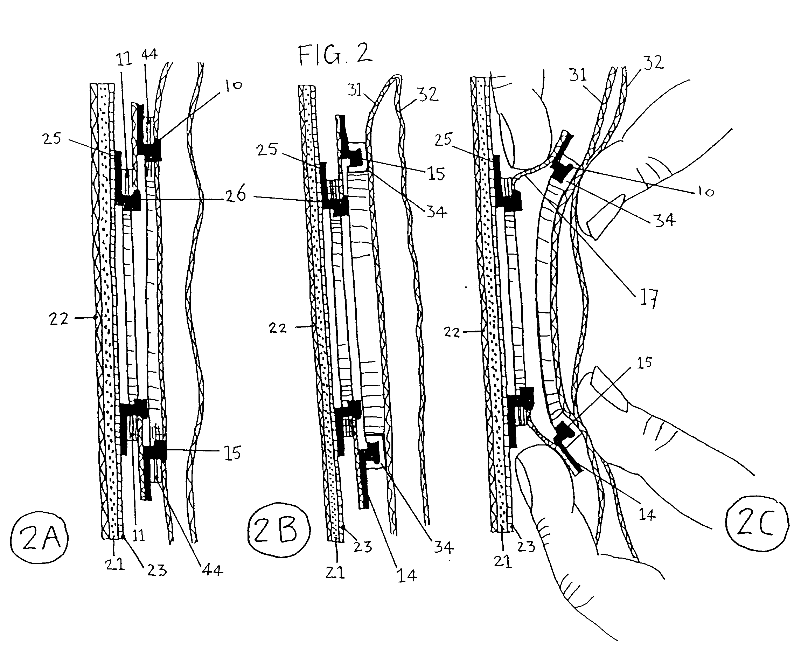

Fig. 2. Shows a cross section of the adapter in different situations. In fig. 2A the adapter has been attached on the body side flange by pushing the smallest flat coupling member (11) around the outer wall of projecting rib (26) of the body side wafer flange, and subsequently on to the larger coupling member of this adapter, a stoma pouch has been attached, by pushing the aperture in the flat coupling member of stoma pouch (40) around the outer wall of projecting rib (15) on the largest coupling member of the adapter (10).

In fig. 2B the same cross section is shown as in fig. 2A, but now another type of pouch has been attached, provided with a channel shaped coupling member (34), which walls are snapped over the projecting rib (15) of the larger coupling member of the same adapter (10).

In fig. 2C is shown how the adapter works in practice. The pouch - also shown in fig. 2B - is going to be attached by snapping the channel of coupling member (34) of the pouch over the projecting rib (15), whereby one can insert the finger tips beneath the flexible wall (17) and thus press both coupling members together between the finger tips and thumbs, without causing any pressures on the peristomal skin.

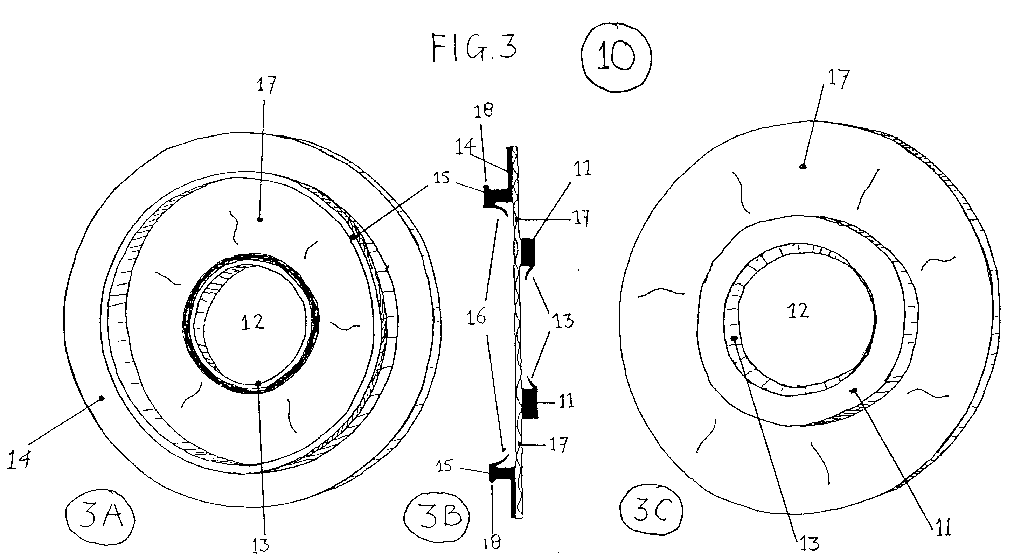

Fig. 3 shows adapter (10) alone. In fig. 3A this adapter is seen from the top. In fig. 3 B a cross section is shown and in fig. 3C one can see the adapter from the bottom. Adapter (10), as shown most prominent in fig. 1 and fig. 3 on one side includes a coupling member (11), consisting of a flat circular shaped disk with a round aperture (12) in the centre, which inner circumference preferably has been completed with a thin flexible and deflectable inwardly extending seal strip (13), in order to make the connection airtight and leak proof. On the opposite side of the adapter (10) a flange (14) has been attached which includes a circular outwardly projected rib (15) which preferably is provided with a thin flexible and deflectable seal strip (16), which extends inwardly from rib (15) in order to create an airtight and leak proof connection with a channel shaped coupling member of a stoma pouch.

The two coupling members of the adapter are joined together by a continuous relatively thin flexible polymeric wall (17). Preferably this continuous wall (17) is flat, but it could also be thermal formed, so that it will include one or more accordion like folds.

Adapter (10) is assembled by a flat circular shaped coupling member (11) with a deflectable seal strip (13) and by a coupling member consisting of a flange (14) with an upstanding or projecting rib (15). Preferably these coupling members are injecting moulded from polyethylene, ethylenevinylacetate copolymer (EVA), a related kind of polymer or from a blend of these polymers.

The continuous wall (17) is made from a straight polymeric film, preferably with a thickness of 75 - 200 micron, preferably produced from polyethylene, EVA, a related kind of polymer or from a blend of these polymers. This continuous wall (17) is attached to both coupling members (11) and (14) by means of heat welding, ultrasonic welding, HF welding or any other suitable bonding.

Adapter (10) is meant for use with a 2-piece stoma system. The body side wafer (20) from this 2-piece system, shown in figs.1 and 2, consists of a layer of pressure sensitive adhesive (21).This adhesive (21) preferably consist of one or more soluble or swellable hydrocolloids dispersed in a visco-elastic fluid such as polyisobutylene, and optionally reinforced with synthetic rubbers, but any known adhesive barrier material is suitable. This adhesive (21) may be picture framed by an extra, adhesive border made from a microporous or non-woven medical plaster with a medical adhesive. The bottom of the adhesive layer (21) is covered by a protective silicone coated release paper or film (22) and the top is covered by a thin polymeric film or foam (23), preferably consisting of polyethylene, EVA, polyurethane, or from a blend of these polymers. The body side adhesive wafer (20) has a centre hole (24) which can be enlarged by the stoma patient by cutting it out to the right stoma size. Flange (25) has been welded or bonded to the polymeric top film (23) of the body side wafer and includes a circular projecting rib type coupling member (26), which inner circumference preferably has been completed with an inward extending thin flexible and deflectable seal strip (27). The stoma pouches (30) and (40) of the 2-piece stoma system each consist of two thin flexible polymeric walls respectively (31), (32) and (41), (42) which are joined together with their outer edges by means of thermal or HF welding. These pouches could be of the colostomy (closed) type, the ileostomy type (with drain outlet and tail clip) or the urostomy type (with drain tap). The coupling member of pouch (30) looks very similar as the one from the earlier mentioned patent application of Steer et al. and thus is not new. The pouch wall (31) of pouch (30) has been provided with a channel shaped coupling member (34), which has been permanently affixed by means of a thermal-, HF- or ultrasonic welding technique. At the upper side of this coupling member (34) there is a pull tab (35) to enable detaching the pouch from the body side wafer and it also includes two opposite ears (36) for the attachment of a support belt if required.

The pouch wall (41) of pouch (40) has been provided with a low profile coupling member, consisting of a preferably solid flat circular shaped disk (44) with a round aperture (45) in the centre, whereby from the bottom inner circumference a deflectable seal strip (46) is extending inwardly in order to create an airtight and leak proof coupling. This coupling member (44) also includes a pull tab (47) at the top to detach the pouch from the body side wafer and optionally it also may have two opposite ears (36) for the attachment of a support belt if required.

In use, at first the adapter (10) is attached to body side wafer (20) by pushing the flat circular shaped coupling member (11) with its centred aperture (12) totally over and around rib (26) of the coupling member (25) on body side wafer (20), so that the flexible and deflectable seal strip (13) will be deformed towards the outer wall of rib (26) and thus creating an air tight and leak proof coupling. It is advised to attach the adapter on to the body side wafer, while it is lying on a table prior to use so that the abdomen will not be disturbed.

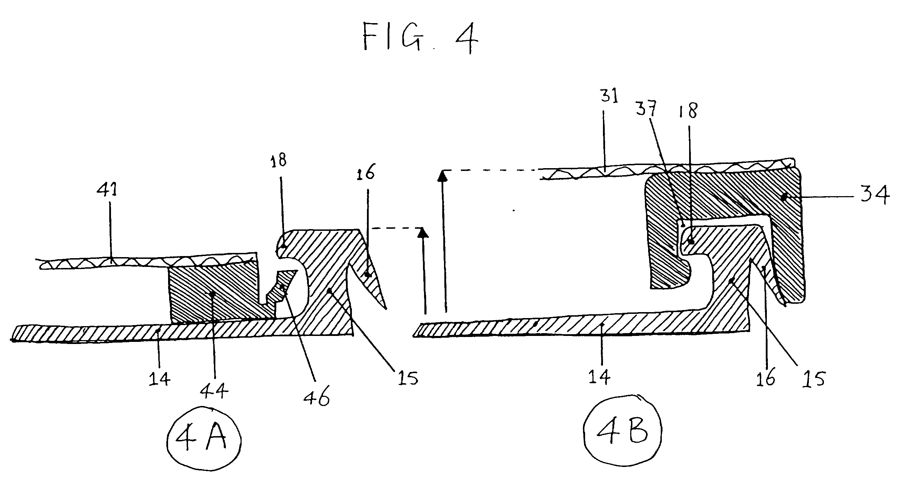

When the aperture in the body side wafer (20) has been cut to size the stoma, the silicone coated release paper or film (22) can be removed and the combination of body side wafer together with the already coupled adapter (10) can be gently adhered to the skin. Regardless whether one prefers a stoma pouch such as type (40) including the low profile coupling member (44) or one prefers a stoma pouch such as type (30) including the widely used channel shaped coupling member (34); both types of coupling members can easily be attached on to or around rib (15) of the largest coupling member (14) of the adapter (10), by pressing both coupling members together between the finger tips and thumbs, as shown in fig. 2C. How these different coupling systems work in practice is shown in a blown up cross section in fig. 4. In both fig. 4A and fig. 4B exactly the same projecting coupling rib (15) on flange (14) is shown, in this case in conformity with the largest coupling member of the adapter, but it could also represent the projecting rib coupling member (25),(26) of body side wafer (20).

Fig. 4A shows the coupling of a pouch type (40), with a flat preferably solid circular shaped coupling member (44) with a round aperture (45), whereby from the bottom of the inner circumference a deflectable seal strip (46) is extending inwardly. (However this cross section could also well be the smaller coupling member (11) with deflectable seal strip (13) of adapter (10).

When the coupling of these two coupling members has been achieved, coupling member (44) will be jammed between flange (14) and the small ridge (18) of the projected rib (15), whereby the thin deflectable seal strip (46) will be deformed towards the outer wall of rib (15) and thus create an airtight and leak proof coupling.

It is obvious, that the aperture in coupling member (44) has been positioned totally around rib (15) in a way that the level of pouch wall (41) of pouch (40) is even lower than the level of rib (15). When these kinds of coupling members are connected - also shown in fig. 4A - the profile of the coupled system does not increase in height.

In fig. 4B the coupling is shown with a pouch type (30), earlier described by Steer et al., thus provided with a circular channel shaped coupling member (34), including a small gutter (37) to fit the small ridge (18) on top of the outer edge of rib (15). Alongside the inner circumference of rib (15) a thin flexible deflectable seal strip extends inwardly which will be deformed towards the inner wall of the channel in coupling member (34) and thus create an airtight and leak proof coupling. With this kind of coupling system, the two walls of the channel shaped coupling member (34) snap over the rib (15) and in fact will be positioned on top of rib (15), whereby the level of pouch wall (31) of pouch (30) is higher than the level of rib (15).

With the kind of coupling system - shown in fig. 4B the profile of the coupled system does increase in height. Both different coupling systems have their grip on to the small ridge (18) at the top of the outer edge of rib (15).

It will be clear that in reality the coupling members of both coupling systems are connected much more tightly than is apparent in figs. 4A and 4B, whereby both deflectable seal strips (46) and (16) will be much more deformed than is shown in the schematic cross sections of figs. 4A and 4B.

As shown in figs. 2A, 2B and 2C and in figs. 3A and 3B, the diameter of the low profile coupling member (11) of the adapter is smaller than the opposite coupling member consisting of flange (14) with projecting rib (15). When the adapter is attached in between the body side wafer and stoma pouch and it is in its relaxed state, flange (14) will lie down closely to the polymeric top film (23) of the body side wafer (20), whereby the relatively flat profile of the total stoma system will be kept maintained.

This profile is even more flat in case the combination of adapter (10) with pouch type (40) (fig. 2A) will be used, because coupling member (44) is totally positioned around the outer wall of rib (15) of flange (14) and thus does not accumulate the total height. However, that will be the case - as shown in fig. 2B and 2C - whereby a pouch type 30 with a channel shaped coupling member (34) has been snapped over rib (15) on flange (14) and thus will accumulate the total height of the systems profile.

The adapter of this patent application will be especially useful for hospitals and institutions because in the post-operative period the peristomal skin is extremely sensitive for pressure.

However, as a result of the extremely low profile of this adapter and its advantage that it can be cleaned very easily, it also is advantageous for use at home within the community, especially for patients who suffer from a very weak abdominal musculature. Another major advantage for patients who prefer a stoma system which permits the finger tips to be inserted beneath the rib coupling member of the body side wafer, is that one does not need to switch over to another type of 2-piece system. This adapter may be used as an optional accessory, so that the patient will keep confidence in his current system. Moreover for hospitals and institutions, a choice between two totally different coupling systems, which both are sized to fit one and the same type of body side wafer, creates a user-friendly and cost effective stoma system.

## Claims

1. An adapter for use together with a 2-piece stoma system, which consist of a body side adhesive wafer incorporating a polymeric circular shaped flange with an upstanding or projecting rib as a coupling member and stoma pouches which - around the stoma aperture - are provided with a coupling member consisting of a flat, preferably solid circular shaped disk, whereby said adapter is characterised by two opposite coupling members, of which the smaller one is provided with a coupling member consisting of a flat preferably solid circular shaped disk with a round aperture in the centre, sized to fit around the projecting rib coupling member of said body side wafer, and the larger one is provided with a circular shaped coupling member with a projecting rib coupling member, which is sized to fit within the aperture of the flat circular shaped coupling member being part of said stoma pouches, whereby both said coupling members are joined together by a continuous flexible and relatively thin polymeric wall by means of bonding or welding, optionally provided with one or more thermoformed accordion folds.

2. An adapter as in claim 1, characterised by the fact that the inner circumference of the round aperture in the smallest coupling member is provided with an inwardly extending flexible and deflectable seal strip, which - when coupled - will be deformed towards the outer wall of the projecting rib coupling member of the body side wafer and whereby the circular shaped projecting rib coupling member at the opposite side of the adapter includes a circular outward projected rib coupling member, which inner circumference is provided with an inwardly extending thin flexible and deflectable seal strip.

3. An adapter as in claim 2, characterised by the fact that this adapter is extremely universal in use as it has been designed to connect different kinds of stoma pouches provided with two totally different kinds of coupling systems on one and the same type of body side flange, such as stoma pouches with a flat circular shaped preferably solid polymeric disk with a centralised stoma aperture, sized to fit around the outer wall of the projecting rib coupling member of the body side wafer and - on the other hand -stoma pouches provided with a circular channel shaped coupling member, whereby its channel is sized to snap over the projecting rib coupling member of the body side wafer.

4. An adapter as in claim 1, characterised by the fact that the outer diameter of said flat circular coupling member which is designed to fit the projecting rib coupling member of a body side flange is smaller than the inner diameter of said projecting rib coupling member designed to fit the coupling member of a stoma pouch, so that this adapter maintains a relatively low profile in its relaxed state.

5. An adapter as in claim 2, characterised by the fact that the outer diameter of said flat circular coupling member which is designed to fit the projecting rib coupling member of a body side flange is smaller than the inner diameter of said projecting rib coupling member designed to fit the coupling member of a stoma pouch, so that this adapter maintains a relatively low profile in its relaxed state.

6. An adapter as in claim 2, characterised by the fact that the relatively thin continuous polymeric wall, optionally provided with one or more thermoformed accordion folds, has been assembled with both said opposite coupling members by means of bonding, thermal welding, HF-welding or ultrasonic welding techniques.

7. An adapter as in claim 2, characterised by the fact that all parts such as the relatively thin continuous polymeric wall, optionally provided with one or more thermoformed accordion folds, and both the said opposite coupling members have been produced or injection moulded in one run as one integral piece.

8. A 2-piece coupling system for stoma pouches and stoma appliances, characterised by the fact that said stoma pouches and stoma appliances - around its stoma apertures - are provided with a circular shaped coupling member consisting of a flat preferably solid polymeric disk with a round stoma aperture in the centre, which inner circumference is provided with an inwardly extending flexible and deflectable seal strip, whereby the aperture of said coupling member is designed to fit securely around the projecting rib coupling member of a body side flange whereby said deflectable seal strip will be deformed towards the outer wall of the projecting rib coupling member of the body side wafer, thus creating an airtight and leak proof seal.

9. A 2-piece coupling system for stoma pouches as in claim 8, whereby said circular shaped coupling member is not solid, but hollow, optionally reinforced with transverse or concentric ribs.

10. A 2-piece coupling system for stoma pouches as in claim 8, whereby said circular shaped coupling member is provided with a pull tab to detach the pouch from the said projecting rib coupling member and optionally can be provided with two opposite ears to enable the attachment of an additional support belt.
